# EUROPEAN PATENT SPECIFICATION

(11) **EP 3 498 277 B1**
(45) Date of publication and mention of the grant of the patent: **06.10.2021**
(21) Application number: 17839780.8
(22) Date of filing: 08.08.2017
(51) Int. Cl.: A61K 31/427, A61K 31/404, A61P 9/10

(54) **PHARMACEUTICAL COMPOSITION FOR STROKE TREATMENT BASED ON AMPK INHIBITION**
PHARMAZEUTISCHE ZUSAMMENSETZUNG ZUR SCHLAGANFALLBEHANDLUNG AUF BASIS VON AMPK-HEMMUNG
COMPOSITION PHARMACEUTIQUE POUR UN TRAITEMENT DE L'ACCIDENT VASCULAIRE CÉRÉBRAL BASÉ SUR L'INHIBITION DE L'AMPK

(30) Priority: 09.08.2016 KR 20160101093; 03.08.2017 KR 20170098417
(43) Date of publication of application: 19.06.2019
(73) Proprietor: Zincure Corp., Seoul 05006 (KR)
(72) Inventor: KIM, Yang Hee, Seoul 05065 (KR); PARK, Hwang Seo, Seoul 04984 (KR); KOH, Jae Young, Seoul 06348 (KR); EOM, Jae Won, Guri-si Gyeonggi-do 11938 (KR); KIM, Tae Youn, Seoul 05023 (KR); SEO, Bo Ra, Seoul 05531 (KR)
(74) Representative: BCKIP
(86) International application number: PCT/KR2017/008569
(87) International publication number: WO 2018/030762

(56) References cited:
- WO-A1-2009/078586
- WO-A1-2015/167246
- WO-A2-2015/153959
- CN-A- 104 059 060
- KR-A- 20080 056 730
- US-A1- 2009 088 432
- US-A1- 2009 163 545
- US-A1- 2014 031 547
- LI J ET AL: "Adenosine monophosphate activated protein kinase inhibition is protective in both sexes after experimental stroke", NEUROSCIENCE LETTERS, ELSEVIER, AMSTERDAM, NL, vol. 482, no. 1, 20 September 2010 (2010-09-20), pages 62-65, XP027204964, ISSN: 0304-3940 [retrieved on 2010-07-16]
- JAE-WON EOM ET AL: "Identifying New AMP-Activated Protein Kinase Inhibitors That Protect against Ischemic Brain Injury", ACS CHEMICAL NEUROSCIENCE, vol. 10, no. 5, 14 February 2019 (2019-02-14), pages 2345-2354, XP055666338, US ISSN: 1948-7193, DOI: 10.1021/acschemneuro.8b00654

## Description

The present invention relates to a pharmaceutical composition for use in stroke treatment, and more particularly, to a novel pharmaceutical composition for use in stroke treatment based on AMPK inhibitory function.

Stroke refers to local neurological symptoms which are caused by a sudden disorder in cerebral blood flow. Brain is accounted only 2% of an entire body weight, but a blood flow rate supplying to the brain is as high as about 15% of cardiac output while oxygen consumption by the brain approaches 20% of whole body oxygen consumption. In addition, since the brain uses only glucose as an energy source, it undergoes necrosis easily even if energy supply is interrupted for a moment. Therefore, the cerebral blood flow closely relates to brain damage. Brain damage caused by stroke involves diverse toxicity mechanisms such as excitotoxicity, oxidative stress, apoptosis and zinc neurotoxicity, therefore, a drug effective for all of various neurotoxic properties is needed to prevent the brain damage.

Currently, although many studies for treatment of stroke have been performed, specific medicines have yet to be developed. Ischemia-reperfusion injury requires an objective evaluation system such as in vitro models or animal models for drug development and effect evaluation thereof based on different complex neuronal cell death mechanisms. However, methods for assessment of a change in vital signs or side effects of medicine treatment are very restricted in a preclinical study stage, thereby likely resulting in failure due to adverse effects during clinical trials. In particular, many clinical studies regarding NMDA antagonists have been performed, but all efforts were unsuccessful. In this regard, Korean Patent Registration No. 1283416 discloses a neuro-protective method that significantly reduces a size of an infracted part by administering AMPK inhibitor compound C or FAS inhibitor C75 to a mouse, so as to retain functions after stroke or ischemic injury.

WO 2009/078586 A1 discloses a composition for prevention and treatment of cancer containing phenyl-amino-thiazolone derivatives inhibiting activity of protein phosphatases or pharmaceutically acceptable salts thereof as an active ingredient.

US 2009/088432 A1 discloses thiazolinones and oxazolinones and their use as Ptplb Inhibitors.

WO 2015/167246 A1 discloses a pharmaceutical composition and a method for treating stroke on basis of AMPK suppression function.

LI J ET AL, NEUROSCIENCE LETTERS, ELSEVIER, AMSTERDAM, NL, vol. 482, no. 1, ISSN 0304-3940, (20100920), pages 62 - 65, describes that adenosine monophosphate activated protein kinase inhibition is protective in both sexes after experimental stroke.

JAE-WON EOM ET AL, ACS CHEMICAL NEUROSCIENCE, US, (20190214), vol. 10, no. 5, doi:10.1021/acschemneuro.8b00654, ISSN 1948-7193, pages 2345 - 2354, identifies AMP-activated protein kinase inhibitors.

However, the above prior art has proved cerebral nerve protective effects of ischemia model animals only, but does not guarantee therapeutic effects on stroke due to other mechanisms except for ischemia. Therefore, the present invention serves to solve various problems including the aforementioned one, and it is an object of the present invention to provide a new stroke medicine based on effective AMPK inhibition in stroke by a variety of mechanisms.

According to an aspect of the present invention, there is provided a pharmaceutical composition for use in treatment of stroke, including a compound having a structure represented by Formula I below as an active ingredient: 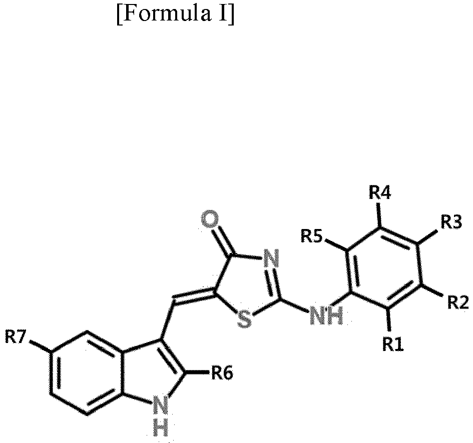 wherein R₁ to R₅ are each independently hydrogen, hydroxyl, halogen, substituted or unsubstituted C₁ to C₇ alkyl, substituted or unsubstituted C₁ to C₇ alkoxy, amine or carboxyl group, or otherwise, R₂ and R₃ together form a -O-(CH₂)ₙ-O- ring or a substituted or unsubstituted benzene ring, wherein n is an integer from 1 to 3; R₆ is hydrogen or a methyl group; and R₇ is hydrogen or a halogen. In the pharmaceutical composition for use in treatment of stroke, the substituted alkyl group is a trifluoromethyl group and the halogen includes iodine, bromine or chlorine.

In accordance with one embodiment of the present invention described above, using a new compound for inhibiting AMPK activity of zinc neurotoxicity as a main cause of stroke may achieve stroke treatment.

FIG. 1 is (A) photographs demonstrating an increase in neurotoxicity after zinc treatment of cultured cerebral cortex neurons identified by TUNEL staining; and (B) and (C) graphs showing a degree of cytotoxicity after treatment using compound C (+Cpd C) as an AMPK inhibitor, which were quantified through TUNEL staining as well as LDH assay (B and C).

FIG. 2 is (A) a Western blot photograph demonstrating an increase in AMPK activity after zinc treatment of cultured cerebral cortex neurons; and (B) an enzymatic activity assay graph.

FIG. 3 is (A) a Western blot photograph demonstrating observation of increased expression of Bim and activity of caspase-3, both being apoptosis promoting genes, after zinc treatment of the cultured cerebral cortex neurons; and (B) a Western blot photograph demonstrating that increases in Bim expression and caspase-3 activity were all attenuated after treatment using compound C (+Cpd C) as an AMPK inhibitor.

FIG. 4 is a graph showing that, after measuring enzymatic activity by AMPK activity assay kit (CycLex, Japan) and a recombinant AMPK (α2/β1/γ1; CycLex, Japan) purchased in the market, and then, comparing the same with effects of compound C, 40 candidate compounds exhibiting similar or stronger inhibition effects were selected.

FIG. 5 is graphs showing observed results for inhibition of different neurotoxicity by inducing such neurotoxicity in the cultured cerebral cortex neurons, and then, using the selected 7 compounds for treatment, followed by quantification of degree of cytotoxicity through LDH assay.

FIG. 6 is graphs showing observation of brain damage inhibitory effects by administering the finally selected drug #28 to an animal model suffering from stroke, and drawings showing comparison results of a degree of final brain damage in the experimental animals compared to a control group.

FIG. 7 is graphs showing results of an acute toxicity test that includes administering the finally selected drug #28 to a rat, and then, extracting a spleen (A), liver (B) and kidney (C).

FIG. 8 is a graph showing observation of neuro-protective effects on zinc neurotoxicity in mouse cortical neuronal cultures by administering 25 similar compounds, which were selected after searching for compounds having a similar structure to the previously selected drug #28.

FIG. 9 is a graph showing observation of neuro-protective effects by selecting 12 drugs exhibiting significant drug effects on zinc toxicity, and then, using the same to treat the mouse cerebral cortex neurons having neuro-toxicity induced by NMDA.

FIG. 10 is a graph showing observation of neuro-protective effects by treating the cerebral cortex neurons of a mouse having neuro-toxicity inducted by H₂O₂ using the selected 12 drugs.

FIG. 11 is graphs showing analyzed results of free zinc concentration by a pZn meter after treatment of neuronal cells using the drug #28 of the present invention and clioquinol at different concentrations.

FIG. 12 is a graph showing analyzed results of a change in free zinc concentrations by applying Newport green DCF (Kd(Zn) = 1µM) as a fluorescent dye after treatment using zinc, clioquinol and the drug #28 on a test tube.

FIG. 13 is a graph showing analyzed results of a change in free zinc concentrations by applying FluoZin-3 (Kd(Zn) = 15nM) as a fluorescent dye after treatment using zinc, clioquinol and the drug #28 on a test tube.

FIG. 14 is (A) a graph showing analyzed results of inhibitory effects of #28 on zinc-mediated neurotoxicity by clioquinol, or pyrithione; and (B) is a graph showing analyzed results of inhibitory effects of #28 or clioquinol as zinc chelator on intracellular zinc-released neurotoxicity by DTDP.

FIG. 15 is (A) photographs demonstrating observation of neuronal cells by a confocal laser microscopy using FluoZin-3 dye after treatment of the neuronal cells using the drug #28; and (B) a graph showing results of quantitative analysis of FluoZin-3 fluorescence magnitude after treatment of 4C01, 4C07, or the drug #28.

FIG. 16 is a graph showing analyzed results of whether neurotoxicity by hydrogen peroxide (H₂O₂) is decreased by a zinc chelator such as TPEN or CaEDTA but not by a calcium chelator such as ZnEDTA, respectively.

FIG. 17 is photographs demonstrating observation of inhibitory effects of TPEN, the drugs #28, 4C01 or 4C07 on hydrogen peroxide (H₂O₂)-mediated intracellular zinc increases by staining with FluoZin-3.

FIG. 18 is a graph showing the protective effect of the drug #28 at different concentrations on neurotoxicity by ionomycin, a calcium ionophore.

Figure 19 is a graph showing analyzed results of chelation effects to calcium using Fura-2 dye as a fluorescent material after treatment using the drug #28 or EDTA on a test tube.

FIG. 20 is (A) a graph showing the inhibitory effect of zinc, the drug #28, or clioquinol on TPEN-induced caspase-3 activity in mouse cortical cultures; and (B) a graph showing attenuation of TPEN-induced neurotoxicity (LDH secretion) by zinc, the drug #28, or clioquinol.

FIG. 21 is a graph showing analyzed results of a change in zinc concentrations within neuronal cells by classifying a neuronal cell culture solution into a plain culture medium, a zinc-supplemented culture medium and a zinc-free culture medium, treating the same with clioquinol and the drug #28 and then applying ZinPyr-1 as a fluorescent material.

As used herein, "AMP-activated protein kinase (AMPK)" is a heterologous trimer protein consisting of a catalytic α subunit (α1 or α2) and two control subunits (β and γ). AMPK is phosphorylated and activated at a low cellular energy level while regulating gene expression over a long period of time by controlling the metabolism of cells, thereby restoring ATP level. It is known that an increase in AMP/ATP ratio, a change in pH of cells and oxidation-reduction state, and an increase in a ratio of creatine/phoscreatine would activate AMPK.

According to an aspect of the present invention, there is provided a pharmaceutical composition for use in treatment of stroke, including a compound represented by formula I below as an active ingredient:

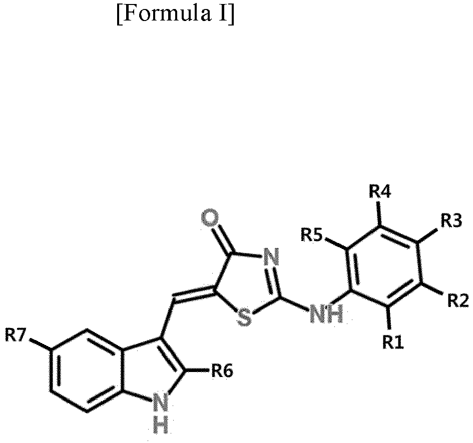

wherein R₁ to R₅ are each independently hydrogen, hydroxyl, halogen, substituted or unsubstituted C₁ to C₇ alkyl, substituted or unsubstituted C₁ to C₇ alkoxy, amine or carboxyl group, or otherwise, R₂ and R₃ together form a -O-(CH₂)ₙ-O- ring or a substituted or unsubstituted benzene ring, wherein n is an integer from 1 to 3; R₆ is hydrogen or a methyl group; and R₇ is hydrogen or a halogen.

In the pharmaceutical composition for use in treatment of stroke, the substituted alkyl group is a trifluoromethyl group and the halogen includes iodine, bromine or chlorine.

In the pharmaceutical composition for use in treatment of stroke, the compound may include, for example, (5Z)-5-(1H-Indol-3-ylmethylene)-2-{[2-(trifluoromethyl)phenyl]amino}-1,3-thiazol-4(5H)-one, (5Z)-5-(1H-Indol-3-ylmethylene)-2-{[3-(trifluoromethyl)phenyl]amino}-1,3-thiazol-4(5H)-one, (5Z)-2-[(3-Bromophenyl)amino]-5-(1H-indol-3-ylmethylene)-1,3-thiazol-4(5H)-one, (5Z)-5-(1H-Indol-3-ylmethylene)-2-[(4-methylphenyl)amino]-1,3-thiazol-4(5H)-one, (5Z)-5-(1H-Indol-3-ylmethylene)-2-[(3-methylphenyl)amino]-1,3-thiazol-4(5H)-one, (5Z)-2-Anilino-5-(1H-indol-3-ylmethylene)-1,3-thiazol-4(5H)-one, (5E)-5-[(2-Methyl-1H-indol-3-yl)methylene]-2-[(4-methylphenyl)amino]-1,3-thiazol-4(5H)-one, 3-{[(5Z)-5-(1H-Indol-3-ylmethylene)-4-oxo-4,5-dihydro-1,3-thiazol-2-yl]amino}benzoic acid, 2-{[(5E)-5-(1H-Indol-3-ylmethylene)-4-oxo-4,5-dihydro-1,3-thiazol-2-yl]amino}benzoic acid, (5Z)-2-[(2-Chlorophenyl)amino]-5-[(2-methyl-1H-indol-3-yl)methylene]-1,3-thiazol-4(5H)-one, or 2-Hydroxy-5-{[(5Z)-5-(1H-indol-3-ylmethylene)-4-oxo-4,5-dihydro-1,3-thiazol-2-yl]amino}benzoic acid.

In the pharmaceutical composition for use in treatment of stroke, the stroke may include, for example, hemorrhagic stroke, ischemic stroke or metal toxicity stroke, wherein the metal may include lead (Pb), mercury, manganese, arsenic, thallium, iron, zinc, cadmium, bismuth or tin. The ischemic stroke may be caused by excitatory neuronal death or oxidative neuronal death.

An effective amount of the compound in the pharmaceutical composition of the present invention may vary depending on types of affected parts of a patient, application site, recovery processing, treatment time, dosage form, patient's conditions, types of adjuvant, etc. The dose is not particularly limited but may range from 0.01 µg/kg/day to 10 mg/kg/day. Dosage per day may include administration once a day, 2 to 3 times a day at an appropriate interval, or intermittent administration at an interval of several days.

In the pharmaceutical composition of the present invention, the effective amount of the compound may range from 0.1 to 100% by weight ('wt.%') based on a total weight of the composition. The pharmaceutical composition of the present invention may further include suitable carriers, excipients and diluents conventionally used for manufacturing a pharmaceutical composition. In addition, the manufacture of the pharmaceutical composition may further include adding an additive for solid or liquid formulations. Such additives for formulation may be either organic or inorganic.

The excipient may include, for example, lactose, sucrose, white soft sugar, glucose, corn starch, starch, talc, sorbit, crystal cellulose, dextrin, kaolin, calcium carbonate, silicon dioxide, etc. The binder may include, for example, polyvinyl alcohol, polyvinyl ether, ethyl cellulose, methyl cellulose, Arabic gum, tragacanth, gelatin, shellac, hydroxypropyl cellulose, hydroxypropyl methyl cellulose, citric acid, calcium, dextrin, pectin, etc. The lubricant may include, for example, magnesium stearate, talc, polyethylene glycol, silica, hardened vegetable oil, etc. The coloring agent may include any one commonly used in the art, provided that it has been permitted as an additive useable for medicines. Tablets or granules thereof may be suitably coated with sugar coating, gelatin coating and other appropriate coatings, as necessary. Further, a preservative, an antioxidant, etc. may also be added, as necessary.

The pharmaceutical composition of the present invention can be manufactured into any formulation that is conventionally prepared in the art (for example, literature [Remington's Pharmaceutical Science, latest edition; Mack Publishing Company, Easton PA]), the type of such preparation is not particularly limited. The formulations have been described in the prescription literature generally known in the pharmaceutical and chemical applications, that is, Remington's Pharmaceutical Science, 15th Edition, 1975, Mack Publishing Company, Easton, Pennsylvania 18042 (Chapter 87: Blaug, Seymour).

The compound in the pharmaceutical composition of the present invention may be orally or topically administered and, preferably, topical (or parenteral) administration including, for example, intravenous injection, subcutaneous injection, intra-cerebroventricular injection, intra-cerebrospinal fluid (CSF) injection, intramuscular injection and intra-peritoneal injection.

The present invention will be described in more detail by the following examples. However, the present invention is not particularly limited to the embodiments set forth herein, instead, may be embodied in many different forms. Therefore, the following examples are provided to more completely describe the present invention.

### General Method.

### Culture of cerebral cortex neurons

Cerebral cortex neurons of a mouse used in the present invention were extracted from the mouse embryos brain and cultured, followed by further culturing the same using a Dulbecco's modified Eagle's medium (DMEM, Gibco, Grand Island, NY, US) containing 5% fetal bovine serum (FBS) and 5% horse serum (HS) under conditions of 95% humidity, 5% CO₂ and 37°C temperature. For activation and differentiation of the above cells, these cells were proliferated at a density of 2x10⁴ cells in 24-well tissue culture plate. Further, prior to treatment using zinc and the compounds, the cells were incubated in MEM medium without FBS and HS.

### Example 1: Determination of reduction in cell toxicity by AMPK inhibitor

According to one embodiment of the present invention, zinc toxicity is one of representative mechanisms causing stroke, and it was investigated whether zinc-toxicity derived in the cultured cerebral cortex neurons is reduced by AMPK inhibitor treatment or not.

More particularly, the cultured cerebral cortex neurons according to the embodiment of the present invention were treated with 300 µM zinc (ZnCl₂) for 10 minutes then removed. 10 hours later, cytotoxicity was observed by TUNEL staining or LDH (Lactate Dehydrogenase). Further, these cells were treated with AMPK inhibitor, that is, 20 µM compound C (+ Cpd C, Tocris), followed by observation whether neurotoxicity is reduced or not.

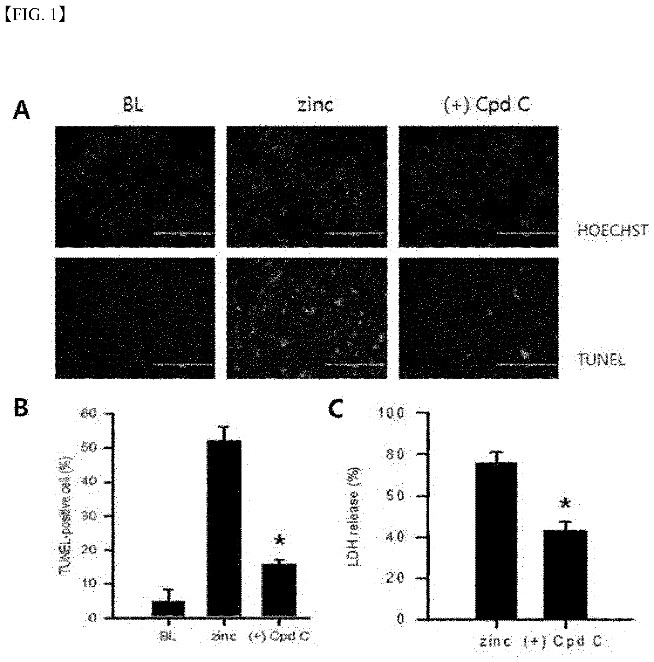

As a result, the cerebral cortex neurons after zinc treatment showed an increase in neurotoxicity, which was demonstrated by TUNEL staining (FIG. 1A). A degree of cytotoxicity was quantified through TUNEL staining and LDH assay. Further, the AMPK inhibitor, that is, compound C (+ Cpd C) demonstrated that the zinc neurotoxicity was significantly reduced (FIGS. 1B and 1C).

### Example 2: Observation of AMPK activity after zinc treatment

In order to understand a correlation between zinc toxicity and AMPK enzymatic activity according to one embodiment of the present invention, the cerebral cortex neurons were treated with zinc, followed by observing AMPK activity through Western blotting and enzymatic activity assays.

### 2-1: Western blot

The cultured cerebral cortex neurons were treated with 300 µM zinc (ZnCl₂) for 10 minutes then removed. After 0.5, 1, 2, 4 and 6 hours, the obtained cell sample was loaded on polyacrylamide gel along with a protein ladder, followed by separation based on a protein size. Thereafter, the sample was treated with an antibody, washed and read out.

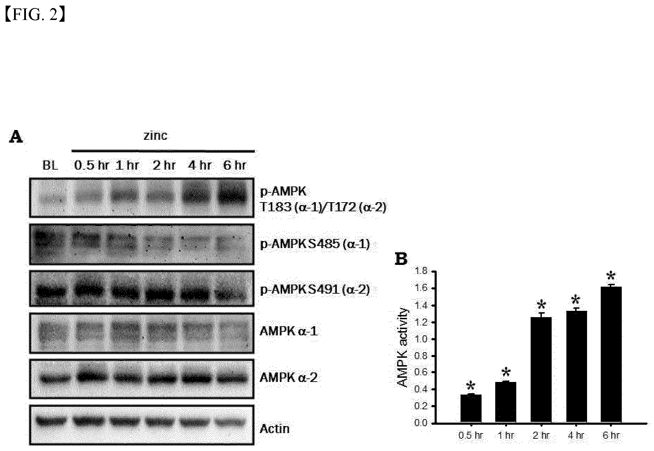

As a result, threonine residues of AMPK alpha-1 and alpha-2 were observed to be phosphorylated. This means AMPK activation, that is, phosphorylated AMPK. However, phosphorylation of other serine residues was not observed (FIG. 2A).

### 2-2: Enzymatic activity assay

Under the same condition as described above, the cerebral cortex neurons were treated with zinc, and protein extracts were obtained at 0.5, 1, 2, 4 and 6 hours after the treatment, followed by measurement of enzymatic activity using an AMPK activity assay kit (CycLex, Japan).

As a result, it was observed that the activity of the AMPK enzyme according to the zinc treatment has a time-dependent increase (FIG. 2B).

### Example 3: Zinc toxicity inhibitory activity through AMPK activity inhibition

According to one embodiment of the present invention, in order to determine whether an increase in enzymatic activity is associated with apoptosis, the cerebral cortex neurons were treated with zinc and observed.

More particularly, the cerebral cortex neurons were treated with 300 µM zinc (ZnCl₂) for 2, 3, 4, 5 and 6 hours to induce zinc toxicity. From each sample, protein was separated and subjected to Western blot and treatment using 20 µM compound C (+ Cpd C) as an AMPK inhibitor, so as to identify a relationship between the zinc toxicity and apoptosis.

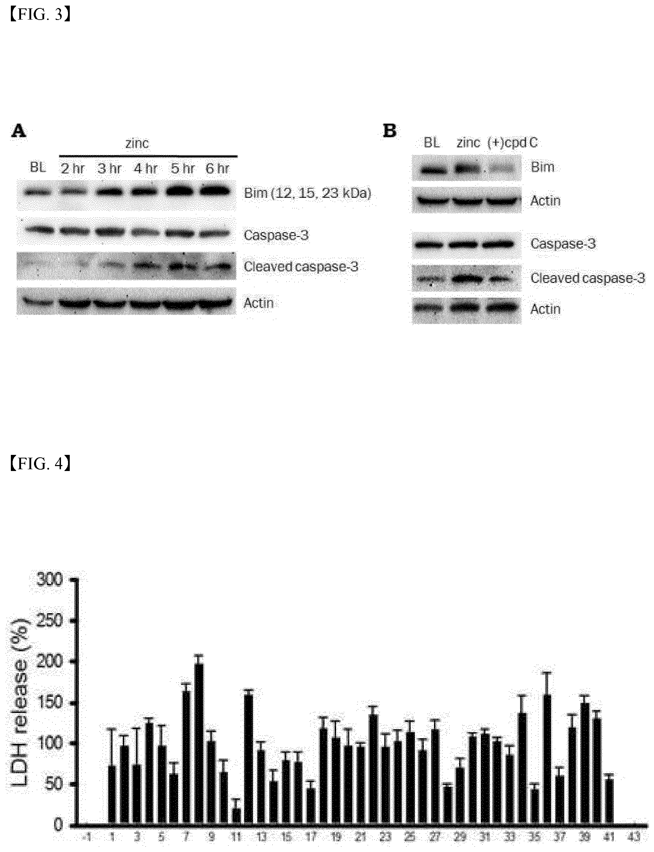

As a result, from 3 hours after the zinc treatment, one in BH3-only Bcl family, which is an apoptosis promoting protein ('pro-apoptotic protein'), that is, Bim showed an increase in expression. Further, cleaved active form of caspase-3 was also observed (FIG. 3A). However, it was found that treatment using an AMPK inhibitor such as compound C can reduce both of the increase in the caspase-3 activity and an increase in Bim caused by zinc toxicity (FIG. 3B). Therefore, it is understood that AMPK enzyme is associated with apoptosis in the zinc toxicity mechanism, and inhibition of the AMPK enzymatic activity means inhibiting apoptosis caused by zinc toxicity.

### Example 4: Screening of AMPK activity inhibitory compound and determination of inhibitory effects

### 4-1: Structure-based virtual screening

With respect to target compounds which may act as an AMPK activity inhibitor, primary screening was carried out according to one embodiment of the present invention.

More particularly, it was identified that the AMPK activity over the prior researches is involved in the zinc toxicity mechanism, and other studies have reported increases in AMPK activity and Bim expression with respect to excitotoxic mechanisms. AMPK enzyme is an enzyme acting with a complex structure of α, β and γ three subunits wherein a double alpha subunit has kinase enzymatic activity. It was reported from results of existing studies that AMPK alpha2 significantly inhibits neurotoxicity caused by ischemia in knock-out mice, contrary to alpha1. Accordingly, the present invention selected candidate chemicals possibly inhibiting AMPK enzymatic activity through structure-based virtual screening with targeting alpha2, resulting in selection of 208 candidate compounds.

### 4-2: AMPK enzymatic activity assay

The 208 candidate compounds selected in Example 4-1 were obtained from a library manufacturer (Interbioscreen, Russia) and were subjected to secondary screening based on observation of AMPK enzymatic activity inhibitory effects thereof.

More particularly, AMPK enzymatic activity was measured by an AMPK activity assay kit (CycLex, Japan) and recombinant AMPK (α2/β1/γ1 CycLex, Japan). As a result of measuring the AMPK enzymatic activity inhibitory effects of the selected 208 compounds as well as existing AMPK inhibitor well known in the art, i.e., compound C, 40 types of drugs at 10 µM exhibited inhibition effects similar to or better than compound C. These 40 drugs are shown in Table 1 below.

**[TABLE 1]**

| Compound ID | Mean (% remaining activity) | SEM | | Compound ID | Mean (% remaining activity) | SEM |
|---|---|---|---|---|---|---|
| #1 | -1.2626 | 1.768 | | #22 | -0.5291 | 0.000 |
| #2 | -1.7934 | 0.072 | | #23 | 1.0582 | 0.000 |
| #3 | -0.9684 | 0.036 | | #24 | 5.0505 | 5.051 |
| #4 | 5.5556 | 0.265 | | #25 | -0.1793 | 0.538 |
| #5 | 4.2328 | 0.529 | | #26 | -1.2554 | 0.036 |
| #6 | 2.3810 | 0.794 | | #27 | 5.8201 | 0.000 |
| #7 | 3.4392 | 0.265 | | #23 | 3.9683 | 1.852 |
| #6 | -0.2152 | 0.215 | | #29 | 0.0000 | 0.000 |
| #9 | 0.5739 | 0.359 | | #30 | 1.0000 | 0.667 |
| #10 | -0.3945 | 0.179 | | #31 | 0.3641 | 0.850 |
| #11 | 5.2910 | 0.529 | | #32 | 0.1257 | 0.126 |
| #12 | -1.3245 | 0.662 | | #33 | 2.9801 | 2.980 |
| #13 | -0.9684 | 0.681 | | #34 | 0.3143 | 0.556 |
| #14 | -0.1435 | 0.000 | | #35 | 0.1214 | 0.121 |
| #15 | 5.0265 | 0.265 | | #36 | 0.2514 | 0.126 |
| #16 | 6.3492 | 0.529 | | #37 | 7.3333 | 1.000 |
| #17 | 2.9101 | 0.265 | | #38 | 5.6667 | 4.667 |
| #18 | 2.9053 | 0.681 | | #39 | 1.667 | 3.333 |
| #19 | 4.3400 | 1.614 | | #40 | 3.6667 | 0.000 |
| #20 | -1.7575 | 0.036 | | Compound C | 5.3000 | 0.760 |
| #21 | 0.8967 | 0.251 | | | | |

### 4-3: Observation of inhibitory effects of the candidate compounds on zinc toxicity

Through observation of inhibitory effects of 40 compounds selected in Example 4-2 above on zinc neurotoxicity, tertiary screening was conducted.

More particularly, the cultured cerebral cortex neurons of a mouse were briefly exposed to 300 µM zinc for 10 minutes, and then treated with the selected 40 drugs, respectively, in order to observe neuro-protective effects. A degree of neuronal death of these cells was quantified by LDH assay wherein effects of individual drugs were averaged to result in a numerical value, and separate experiments were performed on each of the selected drugs 4 times, respectively (FIG. 4).

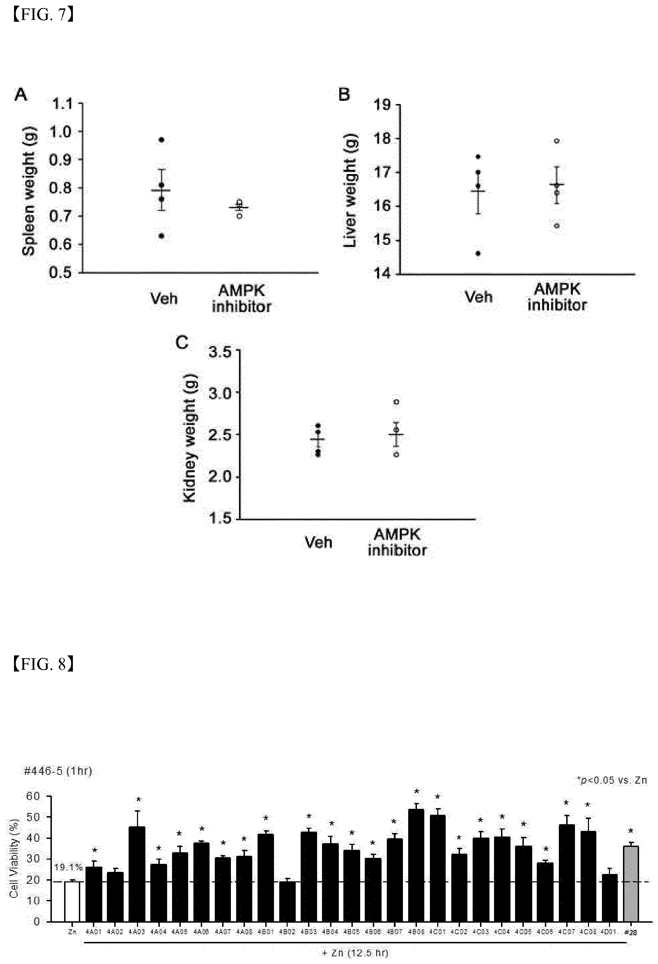

As a result, some compounds inhibited zinc toxicity as shown in FIG. 4, and 7 compounds among those (#6, #11, #14, #17, #28, #35 and #37) were identified to significantly inhibit zinc toxicity as listed in Table 2 below.

**[TABLE 2]**

| Compound ID | Significance | | Compound ID | Significance |
|---|---|---|---|---|
| #1 | 0.5487 | | #22 | 0.0200 |
| #2 | 0.8433 | | #23 | 0.8757 |
| #3 | 0.5597 | | #24 | 0.8699 |
| #4 | 0.0283 | | #25 | 0.4870 |
| #5 | 0.9326 | | #26 | 0.6624 |
| #6 | 0.0278 | | #27 | 0.3030 |
| #7 | 0.0033 | | #28 | 0.0008 |
| #8 | 0.0001 | | #29 | 0.1137 |
| #9 | 0.8649 | | #30 | 0.2142 |
| #10 | 0.1253 | | #31 | 0.1777 |
| #11 | 0.0311 | | #32 | 0.7276 |
| #12 | 0.0000 | | #33 | 0.2386 |
| #13 | 0.4334 | | #34 | 0.1043 |
| #14 | 0.0220 | | #35 | 0.0016 |
| #15 | 0.2378 | | #33 | 0,0381 |
| #13 | 0.2438 | | #37 | 0.0290 |
| #17 | 0.0048 | | #38 | 0.3225 |
| #13 | 0.3961 | | #33 | 0.0027 |
| #19 | 0.7590 | | #40 | 0.0390 |
| #20 | 0.8889 | | Compound C | 0.0003 |
| #21 | 0.6141 | | | |

### 4-4: Observation of inhibitory effects on a variety of neurotoxicity

Through observation of neurotoxicity inhibitory effects of 7 compounds selected in Example 4-3 above, quaternary screening was conducted.

More particularly, the cultured cerebral cortex neurons of a mouse were treated with 50 µM NMDA, 50 µM FeCl₂, 100 µM H₂O₂, 2µM TPEN (N,N,N',N'-tetrakis (2-pyridylmethyl) ethylenediamine, Sigma), 500 nM staurosporine (Abcam) and 10 µM etoposide (Sigma), respectively, to induce neurotoxicity, followed by treatment using the selected 7 compounds at a concentration of 20 µM in order to observe neuro-protective effects. A degree of apoptosis of these cells was quantified by LDH assay. The neurotoxicity models used above include, for example, excitotoxicity, oxidative damage and apoptosis that are considered as a cause mechanism of stroke, in particular, the excitotoxicity model was NMDA, the oxidative damage model was iron toxicity and H₂O₂ toxicity models, and the apoptosis model was TPEN, staurosporine and etoposide toxicity models. The TPEN is a zinc chelator which is well known to cause typical apoptosis in neuronal cells, staurosporine is an enzyme inhibitor (kinase inhibitor) which is also well known as one of typical apoptosis inductive materials, and etoposide is a drug known to cause apoptosis due to DNA damage.

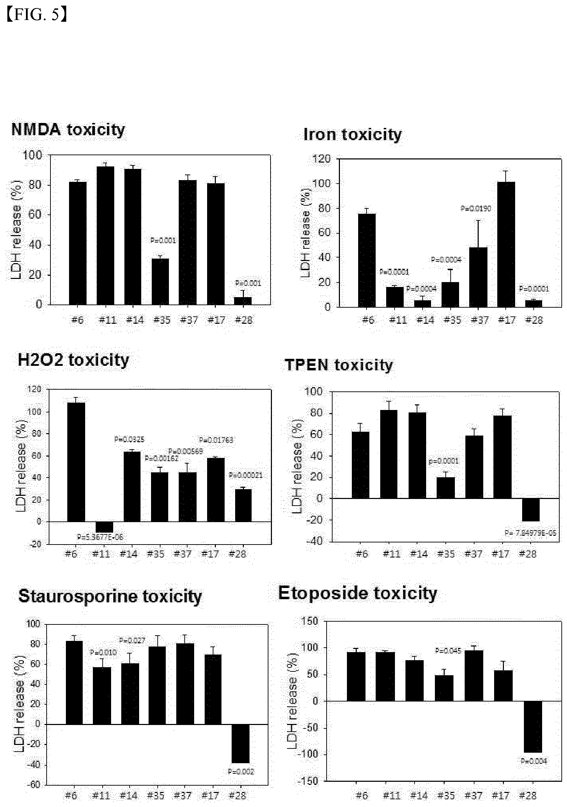

As a result, after treatment using the selected 7 drugs together, neuro-protective effects due to a reduction in neurotoxicity was under observation. In particular, it was found that compound #35 and compound #28 represent inhibitory effects on any toxicity (FIG. 5). The compound #28 obtained from the library purchaser was identified as (Z)-5-((1H-indol-3-yl)methylene)-2-((3-hydroxyphenyl)amino)thiazol-4(5H)-one having the following structure:

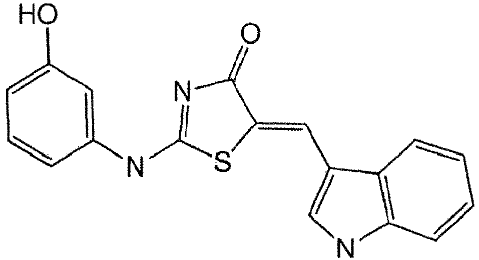

### Example 5: Observation of brain damage inhibitory effects in stroke animal model

In order to determine whether the finally selected compound #28 in Example 4-4 above is effective in practical animal models, a stroke animal model having induced stroke was treated using this compound and then was subjected to investigation for brain damage inhibitory effects.

More particularly, a permanent middle cerebral artery (MCA) occlusion model was prepared using a male Sprague-Dawley (SD) rat aged 8 to 9 weeks. At time points of 30 minutes before MCAO and 10 minutes after the same, cerebral blood flow (CBF) was measured by a laser-doppler flowmeter and the measured results are shown in Table 3 below. Then, 15 minutes after middle cerebral artery occlusion (MCAO), the compound #28, which is finally selected as an AMPK inhibitor (75 ng/3 µl), or a vehicle (10% DMSO) was provided to an area of 0.8 mm on a back and 1.2 mm on a lateral of bregma at a depth of 3.8 mm through intra-cerebroventricular injection. Thereafter, a degree of lack of movement ('motor deficit') was evaluated in the rat, in particular, standards for evaluation are classified as follows (Longa et al., 1989): no deficiency (normal); failure of extending the front foots when vertically stopping (mild); rotation on the opposite side (moderate); and rotation loss (loss of circling) or loss of reflex motion (serious deficit). After induction of ischemia in the rat model, the brain was obtained at the time of lapse of 24 hours, followed by determining a level of cerebral infraction by staining the brain with 2% 2,3,5-triphenyl tetrazolium chloride (TTC).

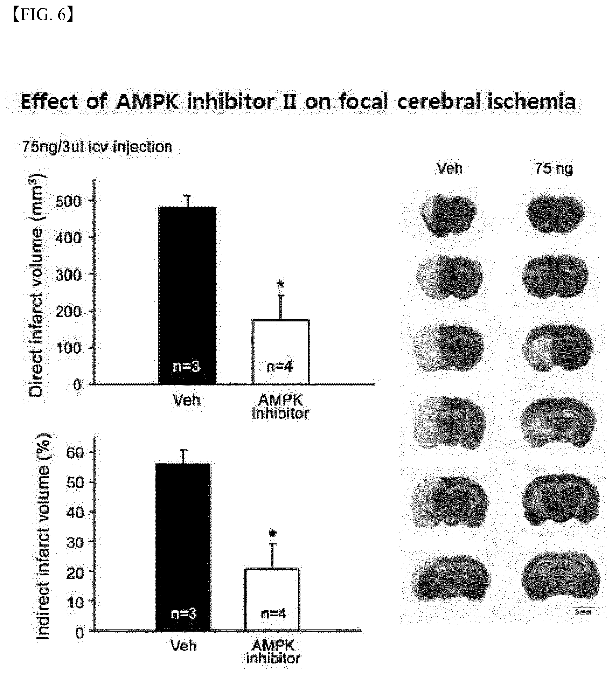

As a result, it was finally identified that compound #28, as the final candidate compound discovered by the present invention, can significantly inhibit brain damage due to permanent middle cerebral artery occlusion (MCAO), which is one of stroke animal models (FIG. 6).

**[TABLE 3]**

| | Veh | AMPK inhibitor |
|---|---|---|
| CBF(% of base line) | 37.8+13.5 | 33.1+9.0 |
| Weight reduction (g) | 48.3+4.7 | 43.8+6.1 |

### Example 6: Acute toxicity test

After injecting the finally selected compound, that is, compound #28, acute toxicity results were under observation.

More particularly, the compound #28, which is finally selected as an AMPK inhibitor, or a vehicle (10% DMSO) was provided to male Sprague-Dawley (SD) rats at an amount of 75 µg/kg per rat through intra-cerebroventricular injection. This procedure was repeated 4 times.

As a result, dead rats were not found even after the elapse of 24 hours. Further, the rat was sacrificed, and specific organs such as the liver, spleen and kidney were extracted, followed by measurement of weights thereof (FIG. 7). Further, after blood sampling, some indicators such as WBC, RBC, BUN, AST, ALT, CREA and GLU were monitored, but as compared to those of the control group, most of these indicators were normal and noticeable toxicity was not observed. Results of the indicators are shown in Table 4 below.

**[TABLE 4]**

| | Normal range | Veh (n=4) | AMPK inhibitor (n = 4) |
|---|---|---|---|
| WBCB(x10³ cell/uL) | 6.6 - 12.6 | 11.0 ± 2.5 | 11.6 ± 1.9 |
| RBC(x10³cell/uL) | 6.8 - 9.8 | 6.7 ± 0.4 | 7.4 ± 0.5 |
| BUN(mg/dL) | 5 - 21 | 16.1 ± 2.2 | 17.6 ± 1.8 |
| AST(U/L) | 45.7 - 80.8 | 121.7 ± 34.6 | 142.3 ± 50.7 |
| ALT(U/L) | 17.5 - 30.2 | 47.4 ± 10.1 | 54.5 ± 8.2 |
| CREA(mg/dL) | 0.2 - 0.8 | 0.5 ± 0. 0 | 0.5 ± 0.0 |
| GLU (mg/dL) | 50 - 135 | 174.6 ± 39. 5 | 174.6 ± 18.0 |

### Example 7: Search of similar compounds and observation of zinc toxicity inhibitory effects

Based on structural similarity of the finally selected compound #28 according to one embodiment of the present invention, 25 similar compounds having the similar structure as described above were purchased from InterBioScreen (Russia) and Akos (Germany). Then, in order to induce zinc toxicity in the cultured cerebral cortex neurons of the mouse, the cultured neurons were treated with ZnCl₂ (400 µM) for 10 minutes and, treated with the above selected 25 compounds as well as the existing selected compound #28 (20 µM), followed by observing whether these compounds inhibit cell death or not through cell viability assay (Cell Counting Kit-8, Dojindo). As a result, except for three drugs among the above 25 new compounds (4A02, 4B02 and 4D01), the remaining 22 drugs exhibited neuro-protective effects (FIG. 8). The names and structures of the selected 25 compounds are listed in Table 5 below.

**[TABLE 5]**

| Code name | Structural Formula | IUPAC name |
|---|---|---|
| 4-A01 | 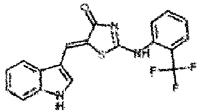 | (5Z)-5-(1H-Indol-3-ylmethylene)-2-{[2-(trifluo romethyl)phenyl]amino}-1,3-thiazol-4(5H)-one |
| 4-A02 | 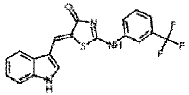 | (5Z)-5-(1H-Indol-3-ylmethylene)-2-{[3-(trifluo romethyl)phenyl]amino}-1,3-thiazol-4(5H)-one |
| 4-A03 | 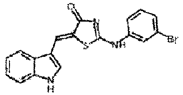 | (5Z)-2-[(3-Bromophenyl)amino]-5-(1H-indol-3-ylmethylene)-1,3-thiazol-4(5H)-one |
| 4-A04 | 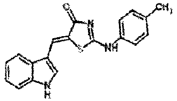 | (5Z)-5-(1H-Indol-3-ylmethylene)-2-[(4-methyl phenyl)amino]-1,3-thiazol-4(5H)-one |
| 4-A05 | 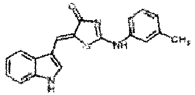 | (5Z)-5-(1H-Indol-3-ylmethylene)-2-[(3-methyl phenyl)amino]-1,3-thiazol-4(5H)-one |
| 4-A06 | 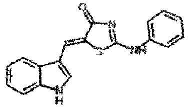 | (5Z)-2-Anilino-5-(1H-indol-3-ylmethylene)-1, 3-thiazol-4(5H)-one |
| 4-A07 | 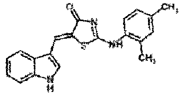 | (5Z)-2-[(2,4-Dimethylpheny])amino]-5-(1H-in dol-3-ylmethylene)-1,3-thiazol-4(5H)-one |
| 4-A08 | 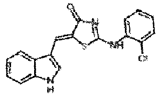 | (5Z)-2-[(2-Chlorophenyl)amino]-5-(1H-indol-3-ylmethylene)-1,3-thiazol-4(5H)-one |
| 4-B01 | 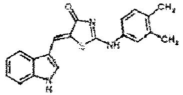 | (5Z)-2-[(3,4-Dimethylphenyl)amino]-5-(1H-in dol-3-ylmethylene)-1,3-thiazol-4(5H)-one |
| 4-B02 | 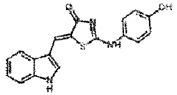 | (5Z)-2-[(4-Hydroxyphenyl)amino]-5-(1H-indol -3-ylmethylene)-1,3-thiazol-4(5H)-one |
| 4-B03 | 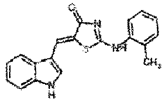 | (5Z)-5-(1H-Indol-3-ylmethylene)-2-[(2-methyl pheny)amino]-1,3-thiazol-4(5H)-one |
| 4-B04 | 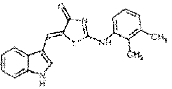 | (5Z)-2-[(2,3-Dimethylphenyl)amino]-5-(1H-in dol-3-ylmethylene)-1,3-thiazol-4(5H)-one |
| 4-B05 | 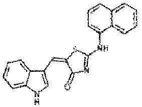 | (5E)-5-(1H-Indol-3-ylmethylene)-2-(1-naphthy lamino)-1,3-thiazol-4(5H)-one |
| 4-B06 | 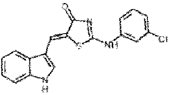 | (5Z)-2-[(3-Chlorophenyl)amino]-5-(1H-indol-3-ylmethylene)-1,3-thiazol-4(5H)-one |

### Example 8: Observation of neurotoxicity inhibitory effect

As a result of repeatedly observing the neuro-protective effects of the 25 similar compounds on zinc toxicity according to one embodiment of the invention, 12 drugs showed continuously significant drug effects. In order to observe different neurotoxicity inhibitory effects, the above 12 drugs were selected and used for the treatment.

More particularly, the neurotoxicity model for observation of neurotoxicity inhibitory effects includes excitotoxicity, oxidative damage, etc. which are classified as a cause mechanism of stroke. NMDA (N-methyl-D-aspartate) was used as the excitotoxicity model while the oxidative model used herein was a hydrogen peroxide (H₂O₂) toxicity model. First, using NMDA (50 µM) or H₂O₂ (100 µM), the cultured cerebral cortex neurons of a mouse were treated for 1.5 hours and 4.5 hours, respectively, resulting in induction of neurotoxicity. The neurons were further treated with the selected 12 drugs (10 µM) as well as the existing selected drug #28 (20 µM). Then, after staining the neurons with propidium iodide (PI), the stained cells were quantified to determine a degree of cell death. The dead cells are stained by PI since they did not have selective permeability in a plasma membrane thereof after the staining, whereas healthy cells are not stained.

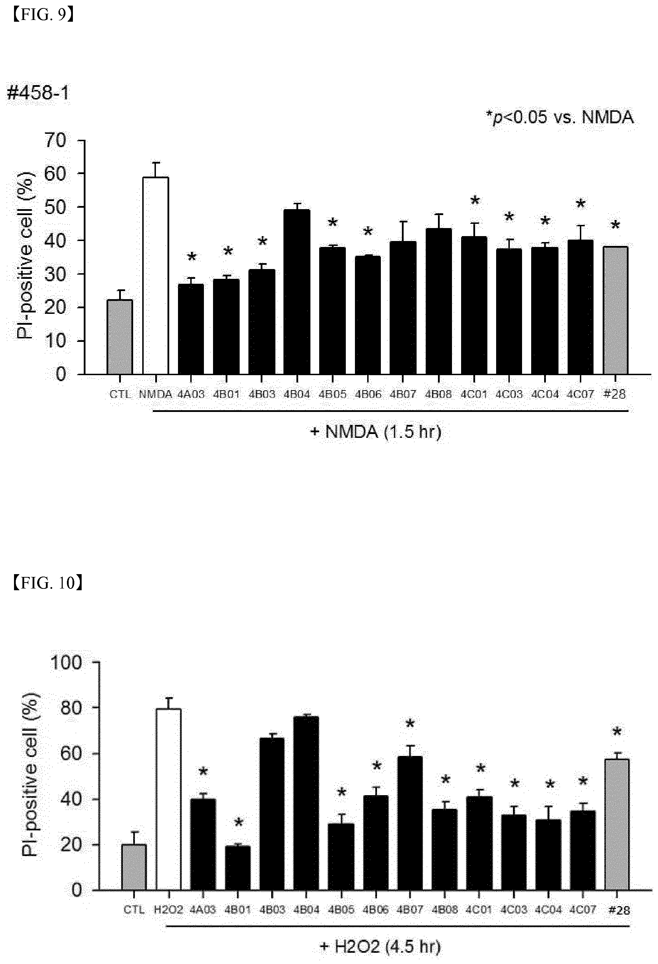

As a result, it was identified that, except for a single drug (4B04, 4B07, 4B08), 9 drugs exhibited neuro-protective effects by significantly inhibiting NMDA-induced neurotoxicity (FIG. 9). Further, 10 drugs other than two drugs (4B03, 4B04) were observed to significantly inhibit H₂O₂-induced neurotoxicity (FIG. 10). These results demonstrated that these drugs have the same or higher neuro-protective effects than the drug #28 selected previously.

### Example 9: Measurement of free zinc concentration

### 9-1: pZn meter

In order to measure free zinc concentration levels, the drug #28 of the present invention was used for treatment in different concentrations (2.5 to 20 µM) along with ZnAF (2.5 µM) as a zinc fluorescent material on a test tube. On the other hand, a control group uses clioquinol (1 to 5 µM) well known as a very stronger zinc chelator. A free zinc concentration was measured by a pZn meter (NeuroBioTex Inc.).

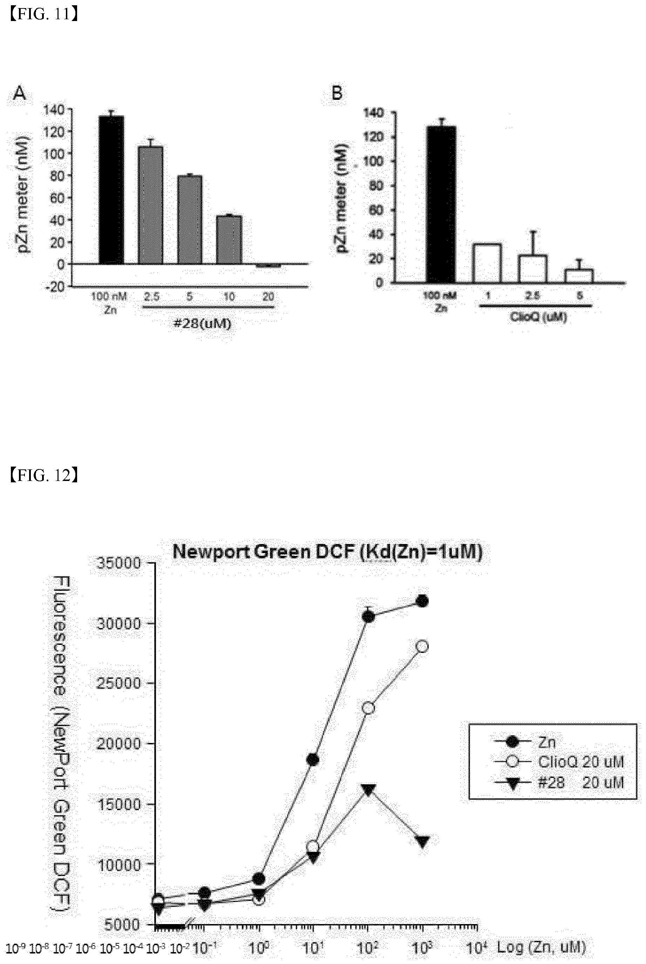

As a result, it was observed that the free zinc concentration was reduced in a concentration-dependent manner by the drug #28 of the present invention (FIG. 11).

### 9-2: Fluorescent dye

Further, in conjunction with clioquinol (20 µM) or the drug #28 (20 µM), a zinc fluorescent dye, i.e., Newport green DCF (0.5 µM, Kd(Zn) = luM) or FluoZin-3 (0.5 µM, Kd(Zn) = 15nM) was used for measurement of free zinc concentration.

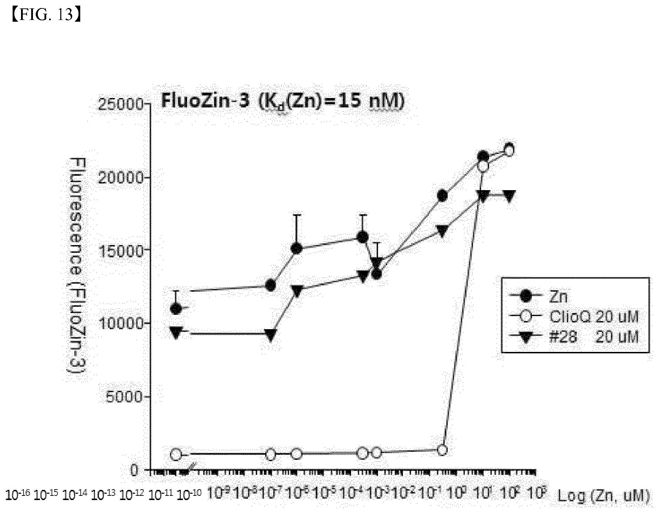

As a result, it was observed that both of the control group, i.e., clioquinol and the drug #28 were combined with zinc ions thus to reduce free zinc ions (FIGS. 12 and 13). In particular, the drug #28 exhibited effects of decreasing the free zinc concentration with respect to high concentration zinc (FIG. 12), however, did not show remarkable effects in a case of low concentration zinc (FIG. 13). These results demonstrated that zinc affinity of the drug #28 is considerably low compared to clioquinol.

### 9-3: Zinc neurotoxicity inhibitory effect

In order to identify that the zinc neurotoxicity inhibitory effects of the drug #28 of the present invention do not influence on a zinc passage channel direct but are a result of zinc chelation, the cultured cerebral cortex neurons of a mouse were treated with clioquinol or pyrithione well known as a zinc ion-permeable carrier ('zinc ionophore') which increases intracellular zinc, as well as a drug inducing intracellular zinc secretion, i.e., (2,2'-Dithiodipyridine), followed by observing neuro-protective effects of the drug #28 on cliquinol, pyrithione, or DTDP-induced neurotoxicity.

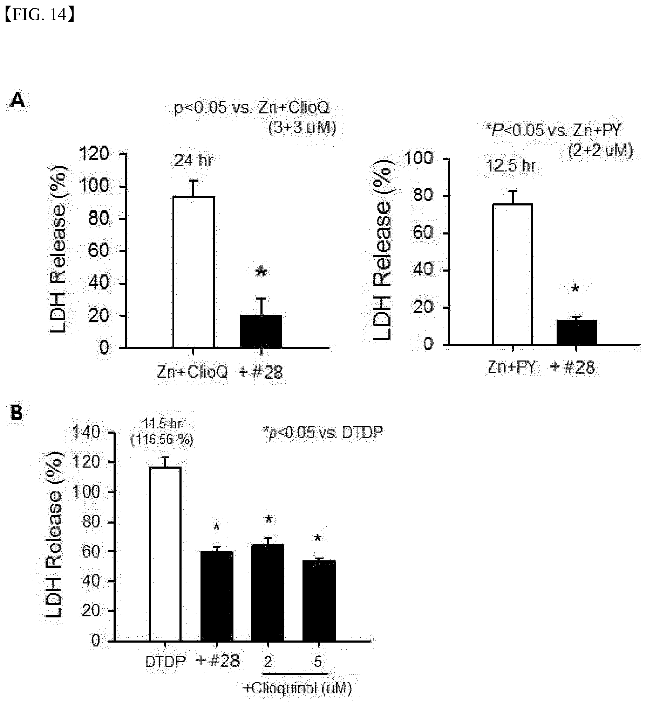

As a result, it was found that zinc neurotoxicity caused by the ion permeable carrier is significantly reduced by the drug #28 (FIG. 14). That is, the effect of the drug #28 may be a result of direct zinc chelation rather than a result of acting on a zinc passage.

### 9-4: Microscope

In order to monitor whether an actual free zinc concentration in neuronal cells is increased or not, the cultured cerebral cortex neurons of a mouse were pre-treated with a FluoZin-3 staining material, exposed to high concentration zinc, and then, treated with 4C01 (20 µM), 4C07 (20 µM) and the drug #28 (20, 50 µM). Thereafter, images of these treated neurons were observed using a confocal laser fluorescence microscope, and a fluorescent size was quantitatively measured using a fluorescence photometer (fluorometer).

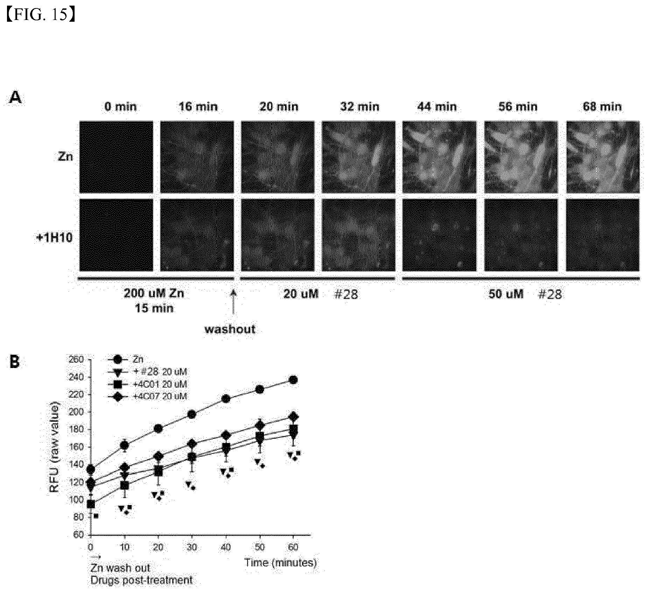

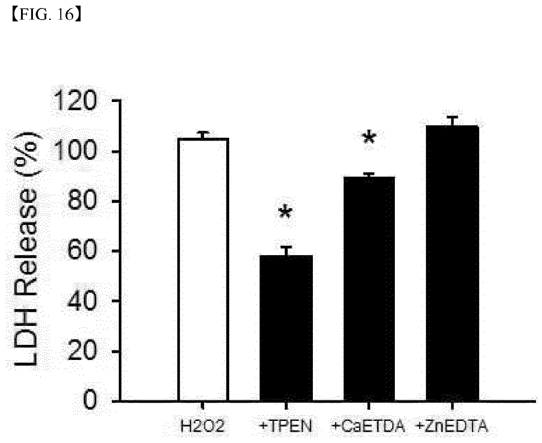

As a result, it was observed that free zinc in the neurons was markedly increased from 16 minutes after the zinc exposure, however, the increase in free zinc in the neurons was significantly reduced by the drug #28 (FIG. 15A). Further, as a result of quantitative analysis of fluorescence magnitude, it was found that not only the drug #28 but also similar compounds 4C01 and 4C07 may also inhibit an increase in intracellular free zinc to the similar level (FIG. 16B).

### 9-5: H₂O₂ toxicity and zinc chelation

There is reported that intracellular free zinc is increased in a neurotoxicity mechanism due to hydrogen peroxide, wherein hydrogen peroxide toxicity is reduced when the cells are treated using the zinc chelator. According to prior experiments, the drug #28 inhibited neurotoxicity due to hydrogen peroxide. Therefore, in order to monitor whether effects of the drug #28 are associated with a reduction of zinc in the above case, the neuronal cells were treated with hydrogen peroxide (H₂O₂), a zinc chelator such as TPEN or CaEDTA and a calcium chelator such as ZnEDTA, followed by monitoring neurotoxicity (LDH secretion). Further, whether free zinc in the neuronal cells is increased or not after hydrogen peroxide treatment was observed through FluoZin-3 staining, and the TPEN treatment group was used as a control group. Other than the drug #28, whether similar compounds 4C01 and 4C07 are under zinc chelation or not was observed by confocal microscopy.

As a result, it was found that TPEN as one of typical zinc chelators reduced neurotoxicity caused by hydrogen peroxide, while another zinc chelator, i.e., CaEDTA also significantly reduced toxicity, thereby demonstrating that H₂O₂ toxicity is associated with zinc. However, EDTA combined with zinc (ZnEDTA) did not inhibit toxicity because EDTA did not further act for chelation of zinc. Consequently, it was found that H₂O₂ neurotoxicity is reduced by zinc chelation (FIG. 16).

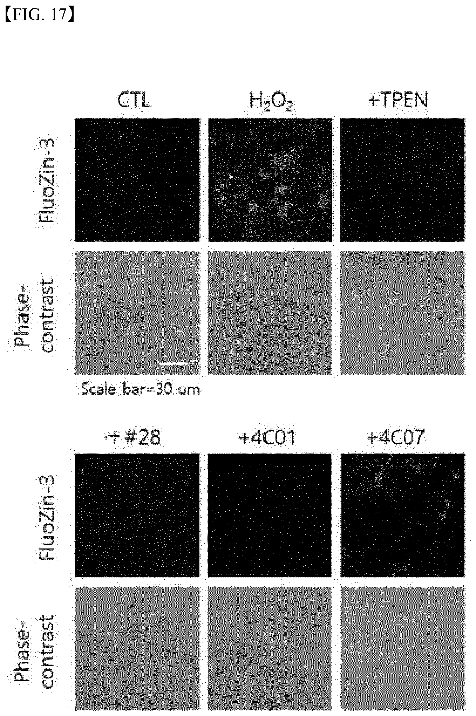

Further, since zinc reduction in the neuronal cells appearing during H₂O₂ toxicity process by treatment using the drugs #28, 4C01 and 4C07 was observed, it is considered that toxicity inhibitory effects achieved by the above drugs is a result of zinc reduction (FIG. 17).

### 9-6: NMDA toxicity and Ca²⁺ chelation

In conventional experiments, excitotoxicity caused by NMDA as a drug for opening an NMDA channel of neuronal cells and flowing calcium ions into the cells was also inhibited by the drug #28. In order to determine whether the above result is obtained by the chelation of Ca²⁺ ions other than the drug #28, protective effects of the drug #28 (10 to 60 µM) after treatment using ionomycin, which is a calcium ion permeable carrier ('calcium ionophore') directly increasing the intracellular calcium concentration, were under observation. Further, using a Fura-2 dye that is combined with calcium to represent fluorescence, it was monitored whether the drug #28 is combined with calcium on a test tube and represents chelating effects.

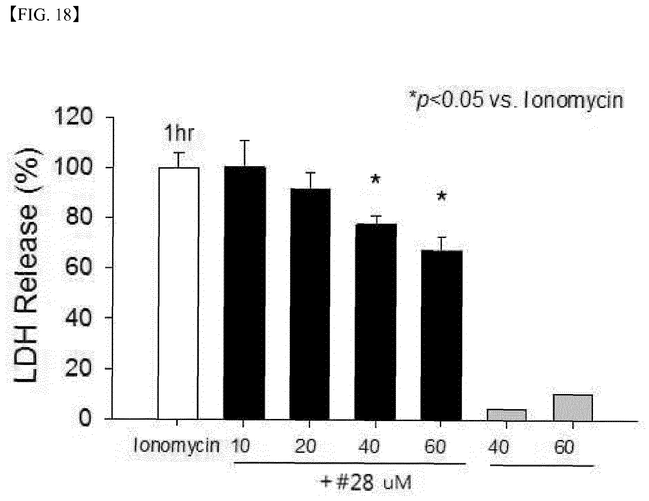

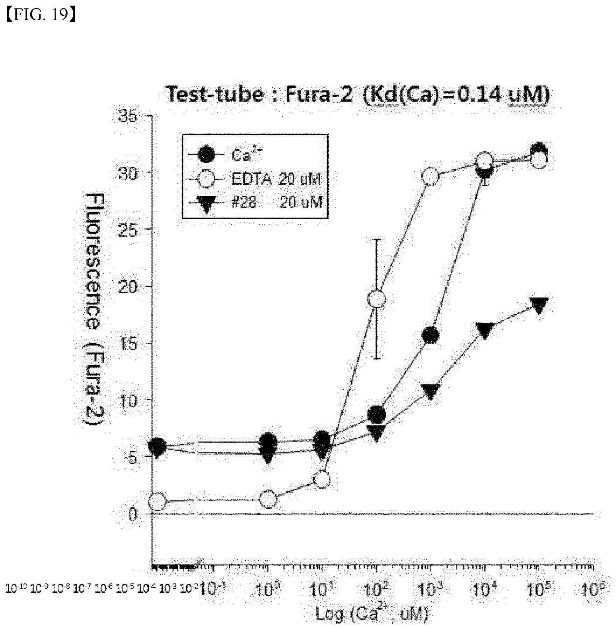

As a result, it was found that treatment using the drug #28 may inhibit toxicity of the ion permeable carrier but the drug has been observed to exhibit toxicity protective effects only when using the same at a concentration of more than 40 µM (FIG. 18). Further, whereas EDTA has good chelating effects at a low calcium concentration, the drug #28 shows the chelating effects for calcium in a high concentration (FIG. 19). Therefore, it can be seen that NMDA neurotoxicity inhibitory effects by the drug #28 are a result of directly chelating calcium ions and have lower affinity to calcium ions than zinc ions.

### 9-6: TPEN toxicity and zinc ion permeable carrier

Generally, application of TPEN as a zinc chelator easily flowing into the cells causes a cell death in the form of typical apoptosis (EDTA is not introduced into the cells). When adding zinc to the above treatment, intracellular free zinc ions are retained thus to reduce neuronal death and further reduce caspase-3 protease activity in the apoptosis process. Under expectation such that: the drug #28 and clioquinol may not only serve as a chelator but also play a role of an ion permeable carrier; these drugs flow into cells as combined with zinc; thereafter, the zinc is removed from the drug because a concentration of free zinc in the cytoplasm is too low; therefore, the concentration of free zinc in the cytoplasm is increased thus to reduce neurotoxicity due to TPEN, caspase-3 activity and neurotoxicity (LDH secretion) were under observation after treatment using zinc, the drug #28 and clioquinol.

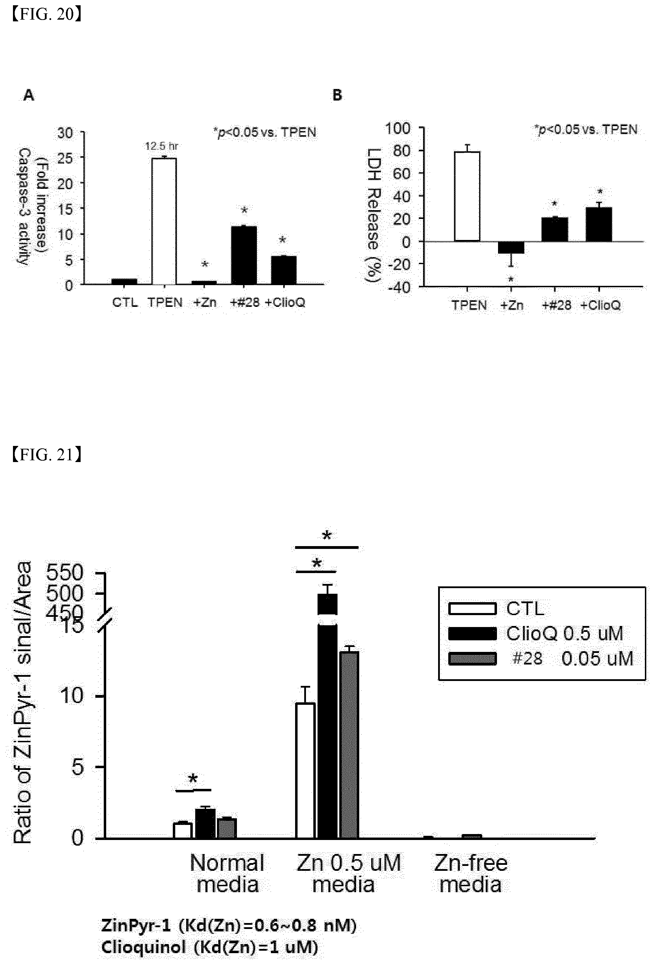

As a result, it was identified that treatment using zinc, the drug #28 and clioquinol can significantly reduce caspase-3 activity induced by TPEN and further decrease neurotoxicity by TPEN (FIG. 20).

### 9-7: Zinc ion permeable carrier

ZinPyr-1 is a fluorescent dye which has a lower Kd value than that of FluoZin-3 and is used to measure a change in a zinc concentration even at a lower concentration. Whether the drug #28 of the present invention can act as an ion permeable carrier was under observation using ZinPyr-1. More particularly, the cultured cerebral cortex neurons of a mouse were treated with ZinPyr-1 then treated with the drug #28 (0.05 µM) and clioquinol (0.5 µM) in a typically used neuronal cell culture medium, so as to determine a change in zinc ion concentrations in the neurons. Also, a non-treatment group was used as a control group.

As a result, under general culture conditions, an increase of zinc by clioquinol was observed, whereas an increase of zinc by the drug #28 was not clearly demonstrated. Therefore, after adding a further 0.5 µM concentration of zinc to the cell culture medium, a change in zinc concentration in the cells was under observation. As a result, significant increase in zinc by treatment using the drug #28 was identified (FIG. 21). Accordingly, it is considered that the drug #28 of the present invention may have a role of an ion permeable carrier and, compared to clioquinol, increase the zinc at a low level.

In fact, clioquinol has high ion affinity and the zinc at a high concentration can be chelated. However, it is well known that, due to a function of the drug itself as an ion permeable carrier, a zinc concentration in cytoplasm is easily increased to cause toxicity. Therefore, clioquinol is not suitable for treatment of stroke but the drug #28 of the present invention may serve as an ion permeable carrier, provided that it does not significantly increase the zinc concentration in cytoplasm. Further, it is considered that, if free zinc in cytoplasm is increased to a level in which toxicity is induced, zinc homeostasis may be controlled to a desired level through chelation.

To summarize the above results, it can be found that AMPK enzyme has an important role on zinc toxicity which is thought as one of possible cause mechanisms for stroke. Therefore, the drug #28 having excellent effects on zinc toxicity was finally selected by screening a new compound candidate group having AMPK activity inhibitory function several times. Further, as a result of treating a stroke animal model with the above drug, it was found that brain damage is significantly reduced. Further, as a result of selecting similar compounds based on a structure of the drug #28, inducing a variety of neurotoxicity and treating the same, these compounds exhibited excellent neuro-protective effects, thereby being applicable as a new stroke treatment agent.

## Claims

1. A pharmaceutical composition for use in stroke treatment, comprising a compound having a structure represented by Formula 1 below as an active ingredient: 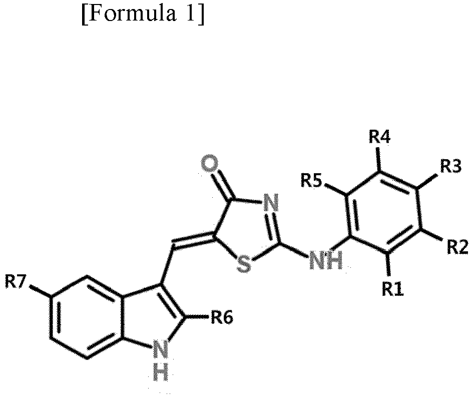 wherein R₁ to R₅ are each independently hydrogen, hydroxyl, halogen, substituted or unsubstituted C₁ to C₇ alkyl, substituted or unsubstituted C₁ to C₇ alkoxy, amine or carboxyl group, or otherwise, R₂ and R₃ together form a -O-(CH₂)ₙ-O- ring or a substituted or unsubstituted benzene ring, wherein n is an integer from 1 to 3; R₆ is hydrogen or a methyl group; and R₇ is hydrogen or a halogen,
the substituted alkyl group is a trifluoromethyl group, and
the halogen includes iodine, bromine or chlorine.

2. The composition for use according to claim 1, wherein the compound is (5Z)-5-(1H-Indol-3-ylmethylene)-2-{[2-(trifluoromethyl)phenyl]amino}-1,3-thiazol-4(5H)-one, (5Z)-5-(1H-Indol-3-ylmethylene)-2-{[3-(trifluoromethyl)phenyl]amino}-1,3-thiazol-4(5H)-one, (5Z)-2-[(3-Bromophenyl)amino]-5-(1H-indol-3-ylmethylene)-1,3-thiazol-4(5H)-one, (5Z)-5-(1H-Indol-3-ylmethylene)-2-[(4-methylphenyl)amino]-1,3-thiazol-4(5H)-one, (5Z)-5-(1H-Indol-3-ylmethylene)-2-[(3-methylphenyl)amino]-1,3-thiazol-4(5H)-one, (5Z)-2-Anilino-5-(1H-indol-3-ylmethylene)-1,3-thiazol-4(5H)-one, (5E)-5-[(2-Methyl-1H-indol-3-yl)methylene]-2-[(4-methylphenyl)amino]-1,3-thiazol-4(5H)-one, 3-{[(5Z)-5-(1H-Indol-3-ylmethylene)-4-oxo-4,5-dihydro-1,3-thiazol-2-yl]amino{benzoic acid, 2-{[(5E)-5-(1H-Indol-3-ylmethylene)-4-oxo-4,5-dihydro-1,3-thiazol-2-yl]amino}benzoic acid, (5Z)-2-[(2-Chlorophenyl)amino]-5-[(2-methyl-1H-indol-3-yl)methylene]-1,3-thiazol-4(5H)-one, or 2-Hydroxy-5-{[(5Z)-5-(1H-indol-3-ylmethylene)-4-oxo-4,5-dihydro-1,3-thiazol-2-yl]amino}benzoic acid.

3. The composition for use according to claim 1, wherein the stroke includes hemorrhagic stroke, ischemic stroke or metal toxicity stroke.

4. The composition for use according to claim 3, wherein the metal includes lead, mercury, manganese, arsenic, thallium, iron, zinc, cadmium, bismuth or tin.

5. The composition for use according to claim 3, wherein the ischemic stroke is derived from excitotoxic neuronal death or oxidative neuronal death.

## Patentansprüche

1. Pharmazeutische Zusammensetzung zur Verwendung in der Schlaganfallbehandlung, umfassend eine Verbindung mit einer Struktur, dargestellt durch die nachstehende Formel 1, als einen aktiven Inhaltsstoff: 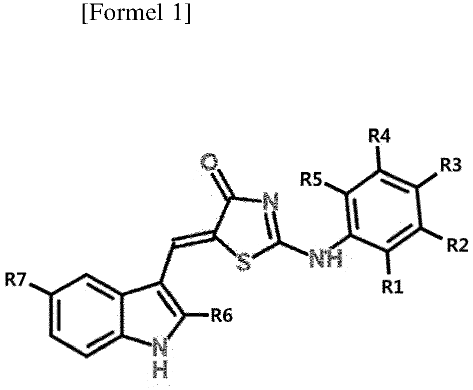 wobei jedes von R₁ bis R₅ unabhängig Wasserstoff, Hydroxyl, Halogen, substituiertes oder unsubstituiertes Ci- bis C₇-Alkyl, substituiertes oder unsubstituiertes Ci- bis C₇-Alkoxy, Amin oder Carboxylgruppe ist oder andernfalls R₂ und R₃ zusammen einen -O-(CH2)ₙ-O-Ring oder einen substituierten oder unsubstituierten Benzolring bilden, wobei n eine ganze Zahl von 1 bis 3 ist; R₆ Wasserstoff oder eine Methylgruppe ist; und R₇ Wasserstoff oder ein Halogen ist,
die substituierte Alkylgruppe eine Trifluormethylgruppe ist, und
das Halogen Iod, Brom oder Chlor einschließt.

2. Zusammensetzung zur Verwendung nach Anspruch 1, wobei die Verbindung (5Z)-5-(1H-Indol-3-ylmethylen)-2-{[2-(trifluormethyl)phenyl]aminl-1,3-thiazol-4(5H)-on, (5Z)-5-(1H-Indol-3-ylmethylen)-2-{[3-(trifluormethyl)phenyl]amin}-1,3-thiazol-4(5H)-on, (5Z)-2-[(3-Bromphenyl)amin]-5-(1H-indol-3-ylmethylen)-1,3-thiazol-4(5H)-on, (5Z)-5-(1H-Indol-3-ylmethylen)-2-[(4-methylphenyl)amin]-1,3-thiazol-4(5H)-on, (5Z)-5-(1H-Indol-3-ylmethylen)-2-[(3-methylphenyl)amin]-1,3-thiazol-4(5H)-on, (5Z)-2-Anilin-5-(1H-indol-3-ylmethylen)-1,3-thiazol-4(5H)-on, (5E)-5-[(2-Methyl-1H-indol-3-yl)methylen]-2-[(4-methylphenyl)amin]-1,3-thiazol-4(5H)-on, 3-{[(5Z)-5-(1H-Indol-3-ylmethylen)-4-oxo-4,5-dihydro-1,3-thiazol-2-yl]amin}benzoesäure, 2-{[(5E)-5-(1H-Indol-3-ylmethylen)-4-oxo-4,5-dihydro-1,3-thiazol-2-yl]amin}benzoesäure, (5Z)-2-[(2-Chlorphenyl)amin]-5-[(2-methyl-1H-indol-3-yl)methylen]-1,3-thiazol-4(5H)-on, oder 2-Hydroxy-5-{ [(5Z)-5-(1H-indol-3-ylmethylen)-4-oxo-4,5-dihydro-1,3-thiazol-2-yl]amin}benzoesäure ist.

3. Zusammensetzung zur Verwendung nach Anspruch 1, wobei der Schlaganfall einen hämorrhagischen Schlaganfall, einen ischämischen Schlaganfall oder einen metalltoxischen Schlaganfall umfasst.

4. Zusammensetzung zur Verwendung nach Anspruch 3, wobei das Metall Blei, Quecksilber, Mangan, Arsen, Thallium, Eisen, Zink, Cadmium, Bismut oder Zinn einschließt.

5. Zusammensetzung zur Verwendung nach Anspruch 3, wobei der ischämische Schlaganfall von exzitotoxischem neuronalem Tod oder oxidativem neuronalem Tod hergeleitet ist.

## Revendications

1. Composition pharmaceutique utilisable pour le traitement d'un accident vasculaire cérébral, comprenant un composé ayant une structure représentée par la formule 1 suivante en tant que principe actif : 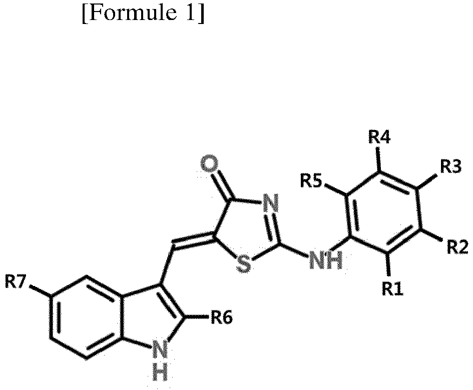 où R₁ à R₅ sont chacun et indépendamment de l'hydrogène, un hydroxyle, un halogène, un alkyle substitué ou non substitué C₁ à C₇, un alcoxy substitué ou non substitué C₁ à C₇, un groupe amine ou carboxyle, ou, sinon, R₂ et R₃ forment ensemble un anneau O-(CH₂)ₙ-O ou un anneau de benzène substitué ou non substitué, n étant un entier entre 1 et 3 ; R₆ est de l'hydrogène ou un groupe méthyle ; et R₇ est de l'hydrogène ou un halogène,
le groupe alkyle substitué est un groupe trifluorométhyle, et
l'halogène comprend l'iode, le brome ou le chlore.

2. Composition utilisable selon la revendication 1, où le composé est (5Z)-5-(1H-3-indolylméthylène)-2-{[2-(trifluorométhyle)phényle]amino}-1,3-4(5H)-thiazolone, (5Z)-5-(1H-3-indolylméthylène)-2-{[3-(trifluorométhyle)phényle]amino}-1,3-4(5H)-thiazolone, (5Z)-2-[(3-bromophényle)amino]-5-(1H-3-indolylméthylène)-1,3-4(5H)-thiazolone, (5Z)-5-(1H-3-indolylméthylène)-2-[(4-méthylephényle)amino]-1,3-4(5H)-thiazolone, (5Z)-5-(1H-3-indolylméthylène)-2-[(3-méthylephényle)amino]-1,3-4(5H)-thiazolone, (5Z)-2-anilino-5-(1H-3-indolylméthylène)-1,3-4(5H)-thiazolone,(5E)-5-[(2-méthyle-1H-3-Indolyl)méthylène]-2-[(4-méthylephényle)amino]-1,3-4(5H)-thiazolone, 3-{[(5Z)-5-(1H-3-indolylméthylène)-4-oxo-4,5-dihydro-1,3-2-thiazolyl]amino} acide benzoïque, 2-{[(5E)-5-(1H-3-indolylméthylène)-4-oxo-4,5-dihydro-1,3-2-thiazolyl]amino} acide benzoïque, (5Z)-2-[(2-chlorophényle)amino]-5-[(2-méthyle-1H-3-indolyl)méthylène]-1,3-4(5H)-thiazolone, ou 2-hydroxy-5-{[(5Z)-5-(1H-3-indolylméthylène)-4-oxo-4,5-dihydro-1,3-2-thiazolyl]amino} acide benzoïque.

3. Composition utilisable selon la revendication 1, où l'accident vasculaire cérébral comprend un accident hémorragique, un accident ischémique cérébral ou une intoxication aux métaux lourds.

4. Composition utilisable selon la revendication 3, où le métal comprend le plomb, le mercure, le manganèse, l'arsenic, le thallium, le fer, le zinc, le cadmium, le bismuth ou l'étain.

5. Composition utilisable selon la revendication 3, où l'accident ischémique cérébral découle d'une mort neuronale excitotoxique ou d'une mort neuronale oxydative.
